**Europäisches Patentamt**

**European Patent Office**

**Office européen des brevets**

(11) Numéro de publication : **0 315 541 B1**

## FASCICULE DE BREVET EUROPEEN

(45) Date de publication du fascicule du brevet :
**22.04.92 Bulletin 92/17**

(51) Int. Cl.$^5$ : **A61K 7/48**

(21) Numéro de dépôt : **88402772.3**

(22) Date de dépôt : **03.11.88**

(54) **Composition cosmétique à base de yaourt ou de kéfir lyophilisé revivifiable.**

(30) Priorité : **05.11.87 FR 8715354**

(43) Date de publication de la demande :
**10.05.89 Bulletin 89/19**

(45) Mention de la délivrance du brevet :
**22.04.92 Bulletin 92/17**

(84) Etats contractants désignés :
**BE CH DE FR GB IT LI NL**

(56) Documents cités :
**FR-A- 2 357 239**
**GB-A- 2 037 160**

(73) Titulaire : **L'OREAL**
**14, Rue Royale**
**F-75008 Paris (FR)**

(72) Inventeur : **Contamin, Jean-Claude**
**9, rue Rodin**
**F-93380 Chilly Mazarin (FR)**
Inventeur : **Meunier, Christine**
**166, rue de Chevilly**
**F-94800 Villejuif (FR)**

(74) Mandataire : **Stalla-Bourdillon, Bernard et al**
**CABINET NONY & CIE 29, rue Cambacérès**
**F-75008 Paris (FR)**

## Description

La présente invention a pour objet une composition cosmétique sous forme de poudre destinée à être hydratée au moment de l'emploi contenant du yaourt ou du kéfir lyophilisé revivifiable ainsi qu'un procédé en vue d'améliorer l'aspect esthétique de la peau, notamment du visage.

Dans le brevet US n°4.268.500 il a été décrit un procédé de traitement de la chevelure en vue de combattre la sécheresse du cuir chevelu et d'améliorer la brillance des cheveux, ce procédé étant essentiellement caractérisé par le fait que l'on applique sur le cuir chevelu et les cheveux du yaourt frais, que l'on couvre le cuir chevelu et les cheveux à l'aide d'un bonnet étanche à l'air, que l'on laisse agir pendant un temps d'environ 30 minutes et que l'on procède ensuite au rinçage du cuir chevelu et des cheveux en vue d'éliminer le yaourt résiduel.

Ce procédé faisant intervenir du yaourt frais est en général mal supporté par les personnes traitées car il provoque, sur le cuir chevelu, des irritations dues à la présence d'acide lactique. Par ailleurs, indépendamment des problèmes de conservation, les yaourts frais ne sont pas toujours d'une consistance appropriée permettant une bonne imprégnation des cheveux et du cuir chevelu.

Dans le brevet britannique n° 2 037 160, il est proposé l'emploi d'un produit lyophilisé dérivé du lait, obtenu à partir de yaourt ayant subi une opération d'hydrolyse enzymatique.

Le lyophylisat est décrit comme contenant une forte teneur en amino acides libres.

Dans le brevet français n°77.20805 (2.357.239) ont été décrites des préparations cosmétiques contenant du yaourt dont la stabilité est obtenue à l'aide de composants stabilisants.

Selon ce brevet, le yaourt peut être du yaourt en poudre ou à l'état frais. Les composants stabilisants sont des agents préservateurs, des anti-oxydants, des agents de chélation (substance formant des complexes métalliques) ainsi que des inhibiteurs de protéinases.

On a toutefois noté que ces compositions, dès leur fabrication, perdaient les propriétés du yaourt ceci étant dû au fait que les agents stabilisants détruisent les ferments lactiques du yaourt.

Les ferments lactiques n'étant plus actifs, on comprend donc que ces compositions qui peuvent être des émulsions solides ou liquides, deviennent dèes lors plus stables dans le temps.

Les bonnes propriétés du yaourt sur la peau étant dues essentiellement à la présence de ferments lactiques, il convient donc que ceux-ci soient toujours actifs lors de l'application.

La présente invention apporte de façon tout à fait originale une solution aux compositions cosmétiques à base de yaourt.

Il est en effet proposé selon la présente invention une composition cosmétique sous forme de poudre destinée à être hydratée au moment de l'emploi contenant du yaourt ou du kéfir lyophilisé revivifiable et au moins un récepteur d'acidité, ladite composition étant exempte de tout agent stabilisant. Une telle composition peut se conserver longtemps sans précaution particulière. Par réhydratation au moment de l'emploi, on revivifie les ferments lactiques et on obtient une composition d'excellente texture qui, appliquée sur la peau, la rend plus lisse et plus douce tout en améliorant sa fermeté.

On retrouve ainsi les propriétés du yaourt frais mais sans en noter les inconvénients, à savoir l'inconfort dû aux rougeurs et aux irritations.

La présente invention a donc pour objet à titre de produit industriel nouveau une composition cosmétique sous forme de poudre destinée à être hydratée au moment de l'emploi, contenant de 50 à 85% en poids de yaourt ou de kéfir lyophilisé revivifiable et au moins un récepteur d'acidité.

Le kéfir ne se différencie du yaourt que par la nature des ferments lactiques et leur nombre.

On rappellera à ce sujet que le yaourt (ou yogourt) est obtenu par ensemencement de lait partiellement ou totalement écrémé, pasteurisé, avec un ferment lactique constitué des bactéries Streptococcus thermophilus et Lactobacillus bulgaricus et incubation à environ 45°C pendant 2 à 3 heures.

Le kéfir (ou kephyr) est obtenu par ensemencement de lait écrémé ou entier avec l'association de bactéries lactiques (Béta bactérium caucasicum en particulier) et d'une levure (Saccharomyces képhyr) et incubation à 25°C une nuit.

Le lyophilisat de yaourt présente les caractéristiques suivantes:

– Lactobacillus bulgaricus: $5{,}6.10^{7}$/g
– Streptococcus thermophilus: $4{,}7.10^{7}$/g
– Coliformes: 0
– Staphylocoques: 0
– Humidité: 6%
– Protéines: 50%
– Lactose: 33%
– Chlorure de sodium: 1,5%

Le lyophilisat de kéfir présente les caractéristiques suivantes:
- Bactéries lactiques (Béta bactérium caucasicum): $2,1.10^3$/g
- Levures (Saccharomyces kephyr): $3,3.10^4$/g
- Coliformes: 0
- Staphylocoques: 0
- Humidité: 7%
- Protéines: 35%
- Lactose: 43%

Le récepteur d'acidité qui lors de la réhydratation permet de neutraliser totalement ou partiellement l'acide lactique résiduel est présent à raison de 0,1 à 40% en poids et de préférence de 0,5 à 35% en poids par rapport au poids total de la composition.

Parmi les récepteurs d'acidité particulièrement préférés selon l'invention, on peut notamment citer l'hydroxyde d'aluminium, l'oxyde de zinc, certaines argiles de type montmorillonite telles que par exemple le "GEL WHITE H-USP" vendu par la Société GREIF, un mélange de dihydrogénophosphate de potassium et d'hydrogénophosphate de dipotassium ou le lait écrémé en poudre.

Selon une forme de réalisation préférée de l'invention, la composition sous forme de poudre peut contenir également un agent de consistance jusqu'à 25% en poids par rapport au poids total de la composition.

Ces agents de consistance sont essentiellement des agents épaississants ou gélifiants qui permettent de conférer à la composition, au moment de sa réhydratation, une consistance appropriée pour une application en couche épaisse homogène.

Parmi les agents de consistance utilisables selon l'invention, on peut notamment citer :
- les gommes de xanthane (polysaccharides) telles que le produit vendu sous la dénomination de "RHODOPOL 23 SC" de la Société RHONE POULENC ou le "KELZAN" de la Société KELCO,
- les dérivés d'algues ou carraghénanes tels que le "SATIAGUM" ou l'"AUBY GUM X 2" de la Société SATIA,
- les alginates tels que le produit vendu sous la dénomination de "SATIALGINE" de la Société SATIA,
- les dérivés de cellulose tels que l'hydroxyméthylcellulose et notamment le produit vendu sous la dénomination de "METHOCEL F 4 M" de la Société DOW CHEMICAL,
- les argiles de la famille des montmorillonites (silicates d'aluminium) telles que la Bentonite USP de la Société GRIEF et,
- les copolymères acryliques sous forme de sel de sodium tels que le produit vendu sous la dénomination de "HOSTACERIN PN 73" de la Société HOECHST.

La composition sous forme de poudre peut également contenir d'autres ingrédients, notamment des substances actives telles que des substances nutritives par exemple des extraits placentaires lyophilisés, du collagène lyophilisé, de la poudre d'avocat ou de la poudre de bardane.

Il convient bien entendu que ces substances soient anhydres pour la bonne stabilité de la composition.

Au moment de l'utilisation, la poudre cosmétique selon l'invention est hydratée par une quantité appropriée d'un soluté liquide qui peut être de l'eau déminéralisée, une eau florale telle que l'eau de camomille, l'eau de bleuet, l'eau de tilleul ou l'eau de menthe, des jus de fruits tels que par exemple ceux de pêche ou d'abricot ou encore des jus de légumes tels que par exemple ceux de concombre ou de carotte.

En vue d'obtenir une bonne consistance, facilitant l'application sur la peau, il est préférable d'utiliser 3 parties de soluté pour 2 parties de poudre, ce rapport variant cependant selon les compositions.

Selon une forme particulière de réalisation le soluté peut également contenir un récepteur d'acidité soluble de préférence un mélange de d'hydrogénophosphate de potassium et d'hydrogénophosphate de dipotassium ce qui permet de stabiliser le pH.

Le soluté liquide peut éventuellement contenir divers ingrédients tels que par exemple un parfum, un colorant, un produit actif hydrosoluble.

Après mélange de la composition cosmétique selon l'invention sous forme de poudre et du soluté liquide, dans un rapport d'environ 1:1 à 1:3 la composition obtenue présente une viscosité comprise entre 10 et 150 poises et de préférence entre 20 et 100 poises et son pH est généralement compris entre 4,5 et 5,5.

Elle peut être, si nécessaire, conservée au froid (+4°C) pendant environ une semaine sans que l'on observe une perte de ses propriétés.

La présente invention a également pour objet un conditionnement en deux parties destiné à être mélangées au moment de l'emploi, caractérisé par le fait qu'il contient au moins une dose de la composition cosmétique sous forme de poudre et au moins une dose correspondante de soluté liquide tel que défini ci-dessus.

De préférence, le coffret contient un nombre de doses suffisant pour un traitement soit d'une semaine, soit de quinze jours à raison d'une application tous les 2 ou 3 jours selon les besoins.

La poudre est généralement conditionnée dans des pots ou des sachets, chacun contenant environ de 4 à 20g de poudre et le soluté liquide dans de petits flacons, chacun contenant de 8 à 30g de soluté.

Au moment de l'emploi, on verse la totalité du soluté dans un récipient contenant la poudre cosmétique et on procède alors au mélange des deux phases jusqu'à l'obtention d'une crème lisse et homogène que l'on peut laisser reposer jusqu'à 60 minutes afin d'obtenir une revivification optimale des ferments.

La présente invention a également pour objet un procédé en vue d'améliorer l'aspect esthétique de la peau, notamment du visage, ce procédé consistant à appliquer sur la peau, sous forme d'une couche épaisse, la crème résultant de la réhydratation de la poudre cosmétique, telle que définie ci-dessus à l'aide du soluté liquide, dans un rapport en poids d'environ 1:1 à 1:3, ladite crème ayant un pH compris entre 4,5 et 5,5, à la laisser agir pendant un temps d'environ 5 à 30 minutes et à procéder ensuite à son élimination par lavage à l'eau ou à l'aide d'un tissu humidifié.

On ne constate au cours du traitement aucun inconfort. Après élimination on observe une amélioration du teint ainsi qu'une plus grande douceur et fermeté de la peau.

On va maintenant donner à titre d'illustration plusieurs exemples de mise en oeuvre de l'invention.

EXEMPLE 1

On prépare selon l'invention une poudre contenant les ingrédients suivants:

```
– Yaourt lyophilisé................ 66,75%
– GEL WHITE H-USP.................. 22,25%
– Lait écrémé en poudre........... 11,00%
```

Après mélange et homogénéisation à l'aide d'un mélangeur, la poudre obtenue est conditionnée dans des pots, chaque pot correspondant à une dose unitaire et contenant 8g de poudre.

Au moment de l'emploi on verse dans un pot, une dose (12g) d'un soluté liquide ayant la composition suivante:

```
– Eau déminéralisée................ 50,00%
– Eau de camomille................ 25,00%
– Eau de tilleul.................. 25,00%
```

Après mélange à l'aide d'une spatule en bois, on obtient une crème lisse et homogène que l'on laisse reposer 30 minutes et que l'on applique ensuite en couche régulière sur le visage préalablement nettoyé. On laisse poser pendant environ 10 minutes puis on élimine la crème à l'aide d'un gant de toilette humide.

On constate que la peau est lisse et douce au toucher.

EXEMPLES 2 à 6

Des effets comparables à ceux décrits ci-dessus ont été obtenus en remplaçant la poudre cosmétique de l'exemple 1 par l'une des poudres des exemples suivants:

EXEMPLE 2

```
– Yaourt lyophilisé................ 81,90%
– Oxyde de zinc................... 17,30%
– Dihydrogénophosphate de potassium  0,20%
– Hydrogénophosphate de dipotassium  0,60%
```

EXEMPLE 3

```
– Yaourt lyophilisé................ 69,50%
– Oxyde de zinc.................... 23,50%
– Lait écrémé en poudre............  7,00%
```

EXEMPLE 4

```
– Yaourt lyophilisé................ 76,60%
– Oxyde de zinc.................... 21,50%
– Carraghénane.....................  1,10%
– Dihydrogénophosphate de potassium 0,20%
– Hydrogénophosphate de dipotassium 0,60%
```

EXEMPLE 5

```
– Yaourt lyophilisé................ 76,60%
– Oxyde de zinc.................... 21,50%
– Gomme de xanthane...............  1,10%
– Dihydrogénophosphate de potassium 0,20%
– Hydrogénophosphate de dipotassium 0,60%
```

EXEMPLE 6

```
– Yaourt lyophilisé................ 55,55%
– Oxyde de zinc.................... 33,45%
– Lait écrémé en poudre........... 11,00%
```

Le soluté liquide de l'exemple 1 peut être remplacé dans les exemples 3 ou 6 par les solutés des exemples A ou C ci-après et dans les exemples 2 à 6 par le soluté de l'exemple B ci-dessous :

EXEMPLE A

```
– Eau de menthe.................... 99,6%
– Dihydrogénophosphate de potassium 0,1%
– Hydrogénophosphate de dipotassium 0,3%
```

EXEMPLE B

```
– Jus de concombre................ 10,00%
– Eau déminéralisée............... 89,70%
– Parfum..........................  0,30%
```

EP 0 315 541 B1

EXEMPLE C

    - Eau de bleuet.................... 20,00%
    - Eau de tilleul................... 20,00%
    - Eau déminéralisée................ 59,40%
    - Dihydrogénophosphate de potassium  0,15%
    - Hydrogénophosphate de dipotassium  0,45%

Tout comme dans l'exemple 1 on constate après application et lavage à l'eau que la peau du visage est plus ferme et plus tendue.

Dans les exemples 1 à 6 ci-dessus le yaourt lyophilisé peut être remplacé par la même quantité de kéfir lyophilisé et l'on observe des résultats tout à fait similaires quant aux effets cosmétiques.

**Revendications**

1. Composition cosmétique sous forme de poudre destinée à être hydratée au moment de l'emploi, caractérisée par le fait qu'elle contient de 50 à 85% en poids de yaourt ou de kéfir lyophilisé revivifiable et au moins un récepteur d'acidité.

2. Composition selon la revendication 1, caractérisée par le fait que le récepteur d'acidité est présent à raison de 0,1 à 40% en poids et de préférence de 0,5 à 35% par rapport au poids total de la composition.

3. Composition selon l'une des revendications 1 et 2, caractérisée par le fait que le récepteur d'acidité est de l'hydroxyde d'aluminium, de l'oxyde de zinc ou une argile de type montmorillonite, un mélange de dihydrogénophosphate de potassium et d' hydrogénophosphate de dipotassium ou du lait écrémé en poudre.

4. Composition selon l'une quelconque des revendications 1 à 3, caractérisée par le fait qu'elle contient en outre un agent de consistance.

5. Composition selon la revendication 4, caractérisée par le fait que l'agent de consistance est présent en une proportion allant jusqu'à 25% en poids par rapport au poids total de la composition.

6. Composition selon l'une quelconque des revendications 4 et 5, caractérisée par le fait que l'agent de consistance est une gomme de xanthane, un dérivé d'algues ou carraghénanes, un alginate, un dérivé de cellulose, une argile de la famille des montmorillonites ou un copolymère acrylique sous forme de sel de sodium.

7. Composition selon l'une quelconque des revendications précédentes, caractérisée par le fait qu'elle contient en outre, une substance active telle qu'un extrait placentaire lyophilisé, du collagène lyophilisé, de la poudre d'avocat ou de la poudre de bardane.

8. Conditionnement en deux parties destinée à être mélangées au moment de l'emploi, caractérisé par le fait que la première partie contient une dose d'une composition cosmétique sous forme de poudre selon l'une quelconque des revendications 1 à 7 et que la deuxième partie contient une dose d'un soluté liquide, ce dernier étant destiné à réhydrater la poudre au moment de l'emploi pour former une crème ayant un pH compris entre 4,5 et 5,5, le rapport en poids de la poudre au soluté liquide étant d'environ 1:1 à 1:3.

9. Conditionnement selon la revendication 8, caractérisé par le fait que le soluté liquide est de l'eau déminéralisée, de l'eau de camomille, de l'eau de bleuet, de l'eau de tilleul ou de l'eau de menthe, un jus de pêche, d'abricot, de concombre ou de carotte.

10. Conditionnement selon la revendication 8, caractérisé par le fait que le soluté contient un récepteur d'acidité soluble, de préférence un mélange de dihydrogénophosphate de potassium et d'hydrogénophosphate de dipotassium.

11. Conditionnement selon l'une des revendications 8 à 10, caractérisé par le fait que le soluté contient en outre un parfum, un colorant ou un produit actif hydrosoluble.

12. Procédé en vue d'améliorer l'aspect esthétique de la peau, notamment du visage, caractérisé par le fait que l'on applique sur la peau, sous forme d'une couche épaisse, une crème obtenue par réhydratation d'une composition cosmétique sous forme de poudre, selon l'une quelconque des revendications 1 7, à l'aide d'un soluté liquide dans un rapport en poids d'environ 1:1 à 1:3, ladite crème ayant un pH compris entre 4,5 et 5,5, que l'on laisse agir pendant un temps d'environ 5 à 30 minutes et qu'on procède ensuite à son élimination par lavage à l'eau ou à l'aide d'un tissu humidifié.

13. Procédé selon la revendication 12, caractérisé par le fait que la crème présente une viscosité comprise entre 10 et 150 poises et de préférence entre 20 et 100 poises.

6

**Patentansprüche**

1. Kosmetiches Mittel in Pulverform, das bei Gebrauch hydratisiert wird, dadurch gekennzeichnet, dass es 50 bis 85 Gew.-% lyophilisierten, reaktivierbaren Joghurt oder Kefir und mindestens einen Säurerezeptor enthält.

2. Mittel nach Anspruch 1, dadurch gekennzeichnet, dass der Säurerezeptor in einer Menge von 0,1 bis 40 Gew.-% und vorzugsweise von 0,5 bis 35 Gew.-%, bezogen auf das Gesamtgewicht des Mittels, vorhanden ist.

3. Mittel nach einem der Ansprüche 1 bis 2, dadurch gekennzeichnet, dass es sich bei dem Säurerezeptor um Aluminiumhydroxid, Zinhoxid oder um Ton vom Montmorillonit-Typ, um eine Mischung von Kaliumdihydrogenphosphat und Dikaliumhydrogenphosphat oder um pulverförmige entrahmte Milch handelt.

4. Mittel nach einem der Ansprüche 1 bis 3, dadurch gekennzeichnet, dass es ausserdem ein Verdickungsmittel enthält.

5. Mittel nach Anspruch 4, dadurch gekennzeichneit, dass das Verdickungsmittel in einer Menge bis zu 25 Gew.-%, bezogen auf das Gesamtgewicht des Mittels, vorhanden ist.

6. Mittel nach einem der Ansprüche 4 und 5, dadurch gekennzeichnet, dass es sich bei dem Verdickungsmittel um Xanthangummi, ein Algen- oder Carrageenan-Derivat, ein Alginat, ein Cellulosederivat, einen Ton vom Montmorillonit-Typ oder ein Acrylcopolymer in der Form des Natriumsalzes handelt.

7. Mittel nach einem der vorhergehenden Ansprüche, dadurch gekennzeichneit, dass es ausserdem eine aktive Substanz, wie einen lyophilisierten Placentaextrakt, lyophilisiertes Collagen, Avocadopulver oder Klettenpulver enthält.

8. Zweiteiliger Behälter, dessen zwei Teile bei Gebrauch gemischt werden, darduch gekennzeichnet, dass der erste Teil eine Dosis eines pulverförmigen kosmetishen Mittel gemäss irgendwelchem Anspruch 1-7 enthält, und dass der zweite Teil eine Dosis einer flüssigen Substanz zur Rehydratisierung des Pulvers bei Gebrauch enthält, wobei eine Crème mit einem pH zwischen 4,5 und 5,5 gebildet wird und das Gewichtsverhältnis zwischen dem Pulver und der flüssigen substanz ca. 1:1 bis 1:3 ist.

9. Zweiteiliger Behälter, nach Anspruch 8, dadurch gekennzeichnet, dass es sich bei der flüssigen Substanz um demineralisiertes Wasser, Kamillenwasser, Kornblumenwasser, Lindenblütenwasser oder Minzewasser, um Pfirsich-, Aprikosen-, Gurken- oder Karottensaft handelt.

10. Zweiteiliger Behälter, nach Anspruch 8, dadurch gekennzeichnet, dass die flüssige Substanz einen löslichen Säurerezeptor, vorzugsweise eine Mischnung von Kaliumdihydrogenphosphat und Dikaliumhydrogenphosphat, enthält.

11. Zweiteiliger Behälter, nach einem der Ansprüche 8 bis 10, dadurch gekennzeichnet, dass die flüssige Substanz ausserden ein Parfum, einen Farbstoff oder ein wasserlösliches aktives Produkt enthält.

12. Verfahren zur Verbesserung des ästhetischen Aussehens der Haut, insbesondere der Gesichtshaut, dadurch gekennzeichnet, dass man auf die Haut eine dicke Schnicht einer Crème aufträgt, welche man durch Rehydratisierung eines pulverförmigen kosmetischen Mittels nach einem der Ansprüche 1 bis 7 mit Hilfe einer flüssigen Substanz in einem Gewichtsverhältnis von ca. 1:1 bis 1:3 erhält, wobei die Crème einen pH zwischen 4,5 und 5,5 hat, dass man die Crème während eines Zeitraums c-von ca. 5 bis 30 Minuten einwirken lässt und man sie anschliessend durch Waschen mit Wasser oder mit Hilfe eines feuchten Tuches entfernt.

13. Verfahren nach Anspruch 12, dadurch gekennzeichnet, dass die Crème eine Viskosität zwischen ca. 10 und 150 P und vorzugsweise zwischen 20 und 100 P hat.


**Claims**

1. Cosmetic composition in the form of a powder intended to be hydrated at the time of use, characterized in that it contains from 50 to 85% by weight of lyophilized revivable yoghurt or kefir and at least one acidity receptor.

2. Composition according to Claim 1, characterized in that the acidity receptor is present in a quantity of 0.1 to 40% by weight, and preferably from 0.5 to 35% with respect to the total weight of the composition.

3. Composition according to one of Claims 1 and 2, characterized in that the acidity receptor is aluminium hydroxide, zinc oxide or a clay of the montmorillonite type, a mixture of potassium dihydrogen phosphate and dipotassium hydrogen phosphate or skimmed milk powder.

4. Composition according to any one of Claims 1 to 3, characterized in that it contains in addition a consistency-improving agent.

5. Composition according to Claim 4, characterized in that the consistency-improving agent is present in a proportion ranging up to 25% by weight with respect to the total weight of the composition.

6. Composition according to either one of Claims 4 and 5, characterized in that the consistency-improving agent is a xanthan gum, a derivative of seaweeds or carraghenans, an alginate, a cellulose derivative, a clay of the montmorillonite family or an acrylic copolymer in the form of a sodium salt.

7. Composition according to any one of the preceding Claims, characterized in that it contains in addition an active substance such as lyophilized placental extract a lyophilized collagen, avocado powder or burdock powder.

8. A two-part packaging to be mixed at the time of use characterized in that the first part contains one dose of a cosmetic composition in powder form according to any one of Claims 1 to 7 and that the second part contains one dose of a liquid solution intended to rehydrate the powder at the time of use to form a cream having a pH of between 4.5 and 5.5, the weight ratio of the powder to the liquid solution being about 1:1 to 1:3.

9. The two-part packaging according to Claim 8, characterized in that the liquid solution is demineralised water, camomile water, cornflower water, lime water or mint water or a peach, apricot, cucumber or carrot juice.

10. The two-part packaging according to Claim 8, characterized in that the solution contains a soluble acidity receptor, preferably a mixture of potassium dihydrogen phosphate and dipotassium hydrogen phosphate.

11. The two-part packaging according to one of Claims 8 to 10, characterized in that the solution contains in addition a fragrance, a colouring or a water-soluble active product.

12. Process for improving the aesthetic appearance of the skin, and of the face in particular, characterized in that a cream obtained by rehydration of a cosmetic composition in the form of a powder according to any one of Claims 1 to 7, with a liquid solution in a weight ratio of about 1:1 to 1:3, the said cream having a pH of between 4.5 and 5.5, is applied to the skin in the form of a thick layer, in that it is left to act for a period of about 5 to 30 minutes and in that it is then removed by washing with water or by using a dampened cloth.

13. Process according to Claim 12, characterized in that the cream has a viscosity of between 10 and 150 poise, and preferably between 20 and 100 poise.